(19) Europäisches Patentamt
European Patent Office
Office européen des brevets

(11) Numéro de publication : **0 495 718 A1**

# (12) DEMANDE DE BREVET EUROPEEN

(21) Numéro de dépôt : **92400112.6**

(51) Int. Cl.$^5$ : **C07C 229/34,** C07D 305/14

(22) Date de dépôt : **16.01.92**

(30) Priorité : **17.01.91 FR 9100491**

(43) Date de publication de la demande : **22.07.92 Bulletin 92/30**

(84) Etats contractants désignés : **PT**

(71) Demandeur : **RHONE-POULENC RORER SA
20, avenue Raymond Aron
F-92160 Antony (FR)**

(72) Inventeur : **Duchesne, Jean-Pierre
160, rue Marcel Mérieux
F-69007 Lyon (FR)**
Inventeur : **Ferraro, Max
1, Chemin Beauregard
F-69320 Feyzin (FR)**

(74) Mandataire : **Pilard, Jacques et al
RHONE-POULENC RORER S.A. 20 Avenue Raymond Aron
F-92165 Antony Cédex (FR)**

## (54) La bêta-Phénylisosérine-(2R,3S), ses sels, sa préparation et son emploi.

(57) La β-phénylisosérine-(2R,3S), ses sels et sa préparation par action de l'ammoniac sur l'acide β-phénylglycidique-(2R,3R) et son emploi pour la préparation de dérivés du taxane de formule générale :

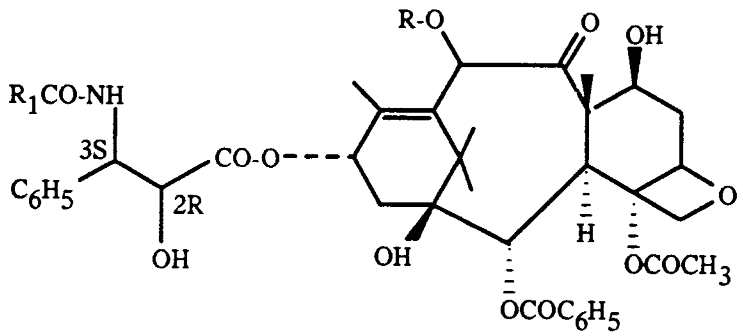

(R = H, $COCH_3$ ; $R_1$ = $C_6H_5$, $(CH_3)_3C$-O-)

EP 0 495 718 A1

La présente invention concerne la β-phénylisosérine-(2R,3S) de formule:

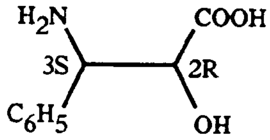

(I)

ainsi que ses sels de métaux alcalins ou alcalino-terreux et ses sels avec les bases azotées, sa préparation et son emploi pour la préparation de produits thérapeutiquement actifs.

Alors que les isomères (2R,3R), (2S,3S) et (2S,3R) de la β-phénylisosérine sont connus dans la littérature scientifique, par exemple dans les articles de E. Kamandi et coll., Arch. Pharmaz., 307. 871-878 (1974), de E. Kamandi et coll., Arch. Pharmaz., 308, 135-141 (1975) ou de K. Harada et Y. Nakajima, Bull. Chem. Soc. Japan, 47, 2911-2912 (1974), la β-phénylisosérine-(2R,3S) ne paraît pas encore avoir été décrite autrement que sous forme d'ester [H. Hönig et coll., Tetrahedron, 46 (11) 3841-3850 (1990)].

Selon l'invention la β-phénylisosérine-(2R,3S), éventuellement sous forme de sel, peut être obtenue par action de l'ammoniaque sur l'acide β-phénylglycidique-(2R,3R) de préférence sous forme de sel alcalin ou alcalino-terreux (sodium, potassium, calcium), de sel d'ammonium ou de sel avec une base azotée (α-méthylbenzylamine, pyridine).

Généralement, le procédé est mis en oeuvre dans l'eau éventuellement en mélange avec un solvant organique tel que le méthanol. De préférence on opère dans l'eau.

Pour la mise en oeuvre du procédé, il est nécessaire d'utiliser un excès d'ammoniac par rapport à l'acide β-phénylglycidique-(2R,3R). Généralement, on utilise de 10 à 100 moles d'ammoniac et de préférence de 50 à 80 moles par mole d'acide β-phénylglycidique-(2R,3R).

L'ammoniac est de préférence utilisé sous forme d'une solution aqueuse concentrée telle qu'une solution dont la concentration est comprise entre 20 et 32 % (p/p) à une température voisine de 25°C.

Le procédé étant mis en oeuvre à une température comprise entre 0 et 100°C, de préférence comprise entre 40 et 60°C. Généralement, on opère sous pression atmosphérique ou bien sous une pression autogène qui est voisine de 2,5 bars à 60 C.

Afin d'accélérer la réaction, il est particulièrement avantageux d'opérer en présence d'un sel d'ammonium tel que le chlorure d'ammonium ou d'hydrogénocarbonate d'ammonium. Il est préférable d'utiliser l'hydrogénocarbonate d'ammonium qui permet d'augmenter la vitesse de réaction tout en conservant la sélectivité. Généralement, on utilise une quantité stoechiométrique de sel d'ammonium par rapport à l'acide β-phénylglycidique mis en oeuvre.

Généralement, le procédé est mis en oeuvre en utilisant le sel de l'acide β-phénylglycidique-(2R,3R) avec l'α-méthylbenzylamine.

Cependant, on peut aussi utiliser un sel de métal alcalin (sodium, potassium) qui est obtenu par action d'une base (soude, potasse) en quantité stoechiométrique sur le sel de l'acide β-phénylglycidique-(2R,3R) avec l'α-méthylbenzylamine, ou le sel d'ammonium qui est obtenu par déplacement du sel de l'acide β-phénylglycidique-(2R,3R) avec l'α-méthylbenzylamine par l'ammoniaque en excès. Dans ce dernier cas, il est possible de favoriser le déplacement par extraction continue ou semi-continue de l'α-méthylbenzylamine au moyen d'un solvant organique convenable tel que le toluène.

Il est particulièrement intéressant d'utiliser le sel d'ammonium de l'acide β-phénylglycidique-(2R,3R) qui permet de rendre l'ouverture au moyen de l'ammoniac à la fois régiosélective et stéréosélective.

Quelle que soit la manière dont l'action de l'ammoniac sur l'acide β-phénylglycidique-(2R,3R) est mise en oeuvre, la β-phénylisosérine-(2R,3S) peut être isolée selon l'une des méthodes suivantes:

1) l'excès d'ammoniac peut être éliminé sous pression réduite de façon à obtenir le sel d'ammonium de l'acide β-phénylglycidique-(2R,3S) en solution aqueuse. Après addition d'un acide minéral fort, la β-phénylisosérine-(2R,3S) précipite et est séparée par filtration, ou bien

2) avant, pendant ou après l'élimination de l'ammoniac sous pression réduite, il est possible d'ajouter une base alcaline (soude, potasse) ou alcalino-terreuse (chaux vive ou éteinte) ; le sel formé précipite après addition éventuelle d'un solvant organique tel que l'acétone. Le sel alcalin ou alcalino-terreux ainsi obtenu est séparé par filtration. Afin de faciliter le relargage du sel, en particulier du sel de sodium, de la β-phénylisosérine-(2R,3S), et d'améliorer le rendement, il peut être avantageux de saturer l'eau présente dans le mélange réactionnel par addition de chlorure de sodium.

L'acide β-phénylglycidique-(2R,3R) peut être préparé dans les conditions décrites par J-N. Denis et al., J. Org. Chem., 51, 46-50 (1986).

La β-phénylisosérine-(2R,3S) obtenue par la mise en oeuvre du procédé selon l'invention est particulièrement utile pour réaliser la synthèse de produits thérapeutiquement actifs tels que les dérivés du taxane de formule générale :

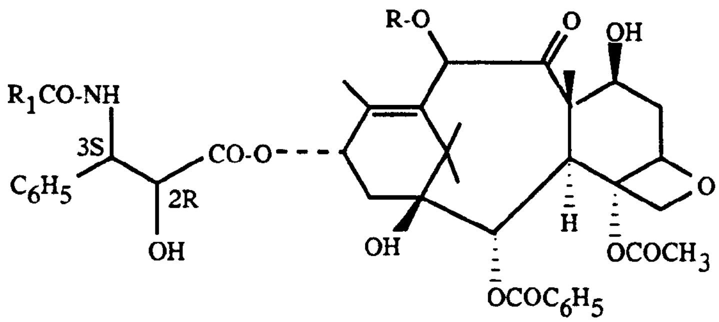

(II)

dans laquelle R représente un atome d'hydrogène ou un radical acétyle et $R_1$ représente un radical phényle ou t.butoxy.

Par action d'un agent de benzoylation (chlorure de benzoyle) ou de t.butoxycarbonylation (dicarbonate de t.butyle) puis d'un agent de protection de la fonction hydroxy, la β-phénylisosérine conduit au produit de formule générale:

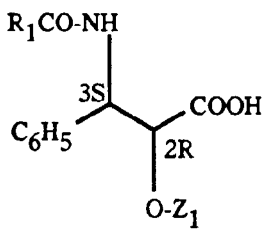

(III)

dans laquelle $R_1$ représente un radical phényle ou t.butoxy et $Z_1$ représente un groupement de la fonction hydroxy (éthoxy-1 éthyle).

Par condensation de l'acide de formule générale (III) sur la baccatine III ou la désacétyl-10 baccatine III dont les fonctions hydroxy en position -7 et éventuellement en position -10 sont protégées par des groupements protecteurs (radicaux silylés, trichloro-2,2,2 éthoxycarbonyle), suivie du remplacement des groupements protecteurs par des atomes d'hydrogène, est obtenu le produit de formule générale (II).

La condensation de l'acide de formule générale (III) sur la baccatine III ou la désacétyl-10 baccatine III protégées ainsi que le remplacement des groupements protecteurs par des atomes d'hydrogène peut être réalisée dans les conditions décrites dans le brevet européen EP-0 336 840 OU EP-0 336 841.

L'exemple suivant, donné à titre non limitatif, montre comment l'invention peut être mise en pratique.

## EXEMPLE 1

Dans une colonne, on introduit 3 kg de β-phénylglycidate-(2R,3R) d'α-méthylbenzylamine, dont le titre est de 98 % et dont l'excès énantiomérique est supérieur à 98,5 %, et 15 litres d'ammoniaque à 32 % (p/p). En bas de colonne, on introduit, au moyen d'une pompe doseuse, du toluène au débit de 3 à 5 litres/heure. La solution toluénique qui décante en tête de colonne est évacuée par débordement. Après avoir introduit 18 litres de toluène, on ne détecte plus d'α-méthylbenzylamine dans l'extrait toluénique.

Dans un autoclave de 150 litres, on introduit la solution de β-phénylglycidate d'ammonium obtenue précédemment et 30 litres d'ammoniaque à 32 % (p/p). L'autoclave est fermé puis chauffé, en 1 heure, à 60°C, sous agitation. La pression est voisine de 2,5 bars. On poursuit l'agitation à 60°C pendant 5 heures puis on laisse refroidir à 18°C. L'ammoniac est éliminé par distillation sous pression réduite (100-700 mm de mercure ; 13,3-93 kPa) à 24°C, après avoir ajouté 9 kg de chlorure de sodium et 0,42 kg de soude en pastilles dans 2,5 litres d'eau. Quand la pression dans l'appareil atteint 45 mm de mercure (6 kPa), le mélange réactionnel est chauffé à 48°C afin de dissoudre les sels puis est refroidi à une température comprise entre -5 et -8°C pendant 3 heures.

Les cristaux blancs obtenus sont séparés par filtration puis séchés à 40°C sous pression réduite (1 mm de mercure; 0,13 kPa). On obtient ainsi 1932 g de sel de sodium de la β-phénylisosérine-(2R,3S) fondant à

218°C.

Le spectre de résonance magnétique nucléaire du $^{13}C$ du sel de sodium de la β-phénylisosérine-(2R,3S) déterminé dans l'eau deutérée à 90 MHz est caractérisé par les déplacements chimiques (δ) suivants: 60,2 ($^{1}JCH$ = 140 Hz) ; 80,0 ($^{1}JCH$ = 147 Hz ; $^{2}J$ = 2,6 Hz); 129,6 ; 130,2 ; 131,5 ; 144,4 et 181,6 ppm.

EXEMPLE 2

Dans un réacteur de 250 litres, on introduit 10 kg de β-phénylglycidate-(2R,3R) d'α-méthylbenzylamine (35,08 moles), 15 litres d'eau et 20 litres de toluène puis on ajoute en 10 minutes 10 litres de soude 4N à une température voisine de 20°C. On agite pendant 1 heure. La phase aqueuse est séparée par décantation. La phase toluénique, qui contient l'α-méthylbenzylamine est conservée. La phase aqueuse est lavée par 2 fois 10 litres de toluène pour éliminer la totalité de α-méthylbenzylamine. Des phases toluéniques réunies peut être isolée l'α-méthylbenzylamine.

A la phase aqueuse, placée dans un réacteur de 250 litres, on ajoute 1,860 kg de chlorure d'ammonium et 162 litres d'ammoniaque à 20 % (p/v). On chauffe à 50°C puis maintient sous agitation pendant 17 heures. Après refroidissement à 35°C on ajoute 35 kg de chlorure de sodium puis maintient à cette température pendant 30 minutes. On laisse refroidir lentement (2 heures) à une température comprise entre 0 et 5°C puis maintient pendant 1 heure à cette température. Le précipité est séparé par filtration puis séché sous pression réduite à 50°C. On obtient ainsi 7 kg de produit sec qui contient environ 25 % de chlorure de sodium et environ 5,300 kg de sel de sodium de la β-phénylisosérine-(2R,3S) pur.

Le rendement est de 72 %.

Le produit ainsi obtenu peut être utilisé tel quel dans les opérations de synthèse ultérieures.

## Revendications

**1 -** La β-phénylisosérine-(2R,3S) ainsi que ses sels de métal alcalin ou alcalino-terreux et ses sels avec les bases azotées.

**2 -** Procédé de préparation de la β-phénylisosérine-(2R,3S) éventuellement sous forme de sel caractérisé en ce que l'on fait agir l'ammoniac sur l'acide β-phénylglycidique-(2R,3R) ou un de ses sels, et isole la β-phénylisosérine-(2R,3S) éventuellement sous forme de sel.

**3 -** Procédé selon la revendication 1 caractérisé en ce que l'on utilise de 10 à 100 moles d'ammoniac par mole d'acide β-phénylglycidique-(2R,3R).

**4 -** Procédé selon l'une des revendications 1 ou 2 caractérisé en ce que l'on opère dans l'eau ou dans un solvant organique.

**5 -** Procédé selon la revendication 4 caractérisé en ce que le solvant est choisi parmi les alcools aliphatiques contenant 1 à 4 atomes de carbone.

**6 -** Procédé selon l'une des revendications 1 à 5 caractérisé en ce que l'on opère, en outre, en présence d'un sel d'ammonium.

**7 -** Procédé selon la revendication 6 caractérisé en ce que le sel d'ammonium est choisi parmi le chlorure d'ammonium et l'hydrogénocarbonate d'ammonium.

**8 -** Procédé selon l'une des revendications 6 ou 7 caractérisé en ce que l'on utilise une mole de sel d'ammonium par mole d'acide β-phénylglycidique-(2R,3R) mis en oeuvre.

**9 -** Procédé selon l'une des revendications 1 à 8 caractérisé en ce que l'on opère à une température comprise entre 0 et 100°C.

**10 -** Utilisation de la β-phénylisosérine-(2R,3S) selon la revendication 1 pour la préparation de dérivés du taxane de formule générale:

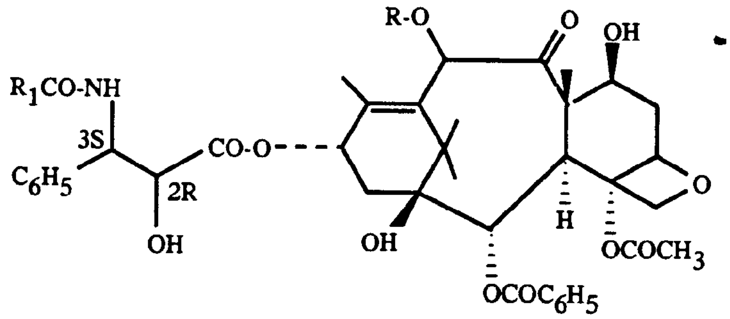

dans laquelle R représente un atome d'hydrogène ou un radical acétyle et $R_1$ représente un radical phényle ou t.butoxy caractérisé en ce que l'on fait réagir un agent de benzoylation ou de t.butoxycarbonylation puis un agent de protection de la fonction hydroxy sur la β-phénylisosérine-(2R,3S), puis condense le produit obtenu sur la baccatine III ou le désacétyl-10 baccatine III dont les fonctions hydroxy en -7 et éventuellement en -10 sont protégées, puis, après remplacement des groupements protecteurs des fonctions hydroxy par des atomes d'hydrogène, isole le produit obtenu.

Office européen des brevets

# RAPPORT DE RECHERCHE EUROPEENNE

Numero de la demande

EP 92 40 0112
Page 1

## DOCUMENTS CONSIDERES COMME PERTINENTS

| Catégorie | Citation du document avec indication, en cas de besoin, des parties pertinentes | Revendication concernée | CLASSEMENT DE LA DEMANDE (Int. Cl.5) |
|---|---|---|---|
| A,D | ARCHIV DER PHARMAZIE.<br>vol. 308, no. 2, Février 1975, WEINHEIM DE<br>pages 135 - 141;<br>E.KAMANDI ET AL: 'DIE SYNTHESE VON BETA-PHENYL-ISOSERINEN DURCH AMMONOLYSE VON BETA-PHENYLGLYCIDESTERN. II.'<br>* le document en entier *<br>--- | 1-9 | C07C229/34<br>C07D305/14 |
| A,D | ARCHIV DER PHARMAZIE.<br>vol. 307, no. 11, Novembre 1974, WEINHEIM DE<br>pages 871 - 878;<br>E. KAMANDI ET AL: 'DIE SYNTHESE VON BETA-PHENYL ISOSERINEN DURCH AMMONOLYSE VON BETA-PHENYL-GLYCIDESTERN. I'<br>* le document en entier *<br>--- | 1-9 | |
| A,D | BULLETIN OF THE CHEMICAL SOCIETY OF JAPAN.<br>vol. 47, Novembre 1974, TOKYO JP<br>pages 2911 - 2912;<br>H. HARADA ET AL: 'OPTICAL RESOLUTION AND CONFIGURATION OF CIS-BETA-PHENYLGLYCIDIC ACID'<br>* le document en entier *<br>--- | 1-9 | **DOMAINES TECHNIQUES RECHERCHES (Int. Cl.5)** |
| A | JOURNAL OF ORGANIC CHEMISTRY.<br>vol. 55, no. 6, 16 Mars 1990, WASHINGTON US<br>pages 1957 - 1959;<br>J.-N. DENIS ET AL: 'AN IMPROVED SYNTHESIS OF THE TAXOL SIDE CHAIN AND OF RP56976'<br>* le document en entier *<br>---<br>-/-- | 10 | C07C |

Le présent rapport a été établi pour toutes les revendications

| Lieu de la recherche | Date d'achèvement de la recherche | Examinateur |
|---|---|---|
| LA HAYE | 03 AVRIL 1992 | SANCHEZ Y GARCIA J. |

**CATEGORIE DES DOCUMENTS CITES**

X : particulièrement pertinent à lui seul
Y : particulièrement pertinent en combinaison avec un autre document de la même catégorie
A : arrière-plan technologique
O : divulgation non-écrite
P : document intercalaire

T : théorie ou principe à la base de l'invention
E : document de brevet antérieur, mais publié à la date de dépôt ou après cette date
D : cité dans la demande
L : cité pour d'autres raisons

& : membre de la même famille, document correspondant

EPO FORM 1503 03.82 (P0402)

Office européen des brevets

# RAPPORT DE RECHERCHE EUROPEENNE

Numero de la demande

EP 92 40 0112
Page 2

## DOCUMENTS CONSIDERES COMME PERTINENTS

| Catégorie | Citation du document avec indication, en cas de besoin, des parties pertinentes | Revendication concernée | CLASSEMENT DE LA DEMANDE (Int. Cl.5 ) |
|---|---|---|---|
| A | CHEMICAL ABSTRACTS, , no. 115,<br>28 Octobre 1991, Columbus, Ohio, US;<br>abstract no. 183831G,<br>page 985 ;<br>* abrégé *<br>& AMINO ACIDS- CHEM.,BIOL.,MED.,(PAP.INT.CONGR. AMINO ACID RES.)<br>vol. 1989, 1990,<br>pages 134 - 142;<br>H HOENIG ET AL: 'CHEMOENZYMIC SYNTHESIS OF ENANTIOMERICALLY PURE HYDROXY AMINO ACIDS'<br>--- | 1-9 | |
| P,X | JOURNAL OF ORGANIC CHEMISTRY.<br>vol. 56, no. 5, 1 Mars 1991, WASHINGTON US<br>pages 1681 - 1683;<br>I. OJIMA ET AL: 'EFFICIENT AND PRACTICAL ASYMMETRIC SYNTHESIS OF THE TAXOL C-13 SIDE CHAIN, N-BENZOYL-(2R,3S)-3-PHENYLISOSERINE, AND ITS ANALOGUES VIA CHIRAL ESTER ENOLATE-IMINE CYCLOCONDENSATION'<br>* le document en entier *<br>----- | 1,10 | |

DOMAINES TECHNIQUES RECHERCHES (Int. Cl.5 )

Le présent rapport a été établi pour toutes les revendications

| Lieu de la recherche | Date d'achèvement de la recherche | Examinateur |
|---|---|---|
| LA HAYE | 03 AVRIL 1992 | SANCHEZ Y GARCIA J. |

**CATEGORIE DES DOCUMENTS CITES**

X : particulièrement pertinent à lui seul
Y : particulièrement pertinent en combinaison avec un autre document de la même catégorie
A : arrière-plan technologique
O : divulgation non-écrite
P : document intercalaire

T : théorie ou principe à la base de l'invention
E : document de brevet antérieur, mais publié à la date de dépôt ou après cette date
D : cité dans la demande
L : cité pour d'autres raisons

& : membre de la même famille, document correspondant

EPO FORM 1503 03.82 (P0402)